# FASCICULE DE BREVET EUROPEEN

(11) **EP 0 881 898 B1**
(45) Date de publication et mention de la délivrance du brevet: **08.01.2003**
(21) Numéro de dépôt: 97913289.1
(22) Date de dépôt: 10.11.1997
(51) Int. Cl.: A61K 7/48, A61K 35/78

(54) **COMPOSITION POUR LA PREVENTION DU VIEILLISSEMENT CUTANE**
MITTEL ZUR VERHINDERUNG DER HAUTALTERUNG
COMPOSITION FOR PREVENTING AGEING OF THE SKIN

(30) Priorité: 14.11.1996 FR 9614121
(43) Date de publication de la demande: 09.12.1998
(73) Titulaire: LANATECH LABORATOIRE NATURE ET TECHNIQUE, 75116 Paris (FR)
(72) Inventeur: VACHER, Anne-Marie, F-78150 Le Chesnay (FR); FRITSCH, Marie-Claire, F-75007 Paris (FR)
(74) Mandataire: de Saint-Palais, Arnaud Marie
(86) Numéro de dépôt international: FR9702017
(87) Numéro de publication internationale: WO98020851

(56) Documents cités:
- DE-A- 2 801 186
- FR-A- 2 618 071
- STN, Serveur de Bases de Données, XP002033663
- STN, Serveur de Bases de Données, XP002056861

## Description

La présente invention concerne une composition servant à la prévention du vieillissement cutané. Elle s'applique tout aussi bien au vieillissement naturel (action anti-âge) qu'aux phénomènes de vieillissement accidentels de la peau dus aux multiples agressions que subit quotidiennement la peau, en particulier, celles dues aux rayonnements solaires (produits solaires et après soleil).

D'une façon générale, on sait que le vieillissement est un phénomène inéluctable mais certains facteurs peuvent en accélérer le processus. Il est connu que les rayonnements ultraviolets de la lumière altèrent les fonctions normales de la peau et que les expositions prolongées aux radiations solaires contribuent au vieillissement et à l'induction de cancers cutanés. Les radicaux libres sont les agents principaux incriminés dans ces altérations.

Les radicaux libres sont des particules agressives qui possèdent un électron libre, d'une grande réactivité chimique, et sont donc particulièrement instables.

Ils sont provoqués par les radiations ionisantes, le rayonnement ultraviolet et même la lumière visible, mais ils sont également issus des réactions métaboliques et enzymatiques qui se produisent dans l'organisme. Ces réactions ont lieu par exemple, lors de la respiration cellulaire, au niveau des mitochondries ou de la synthèse des prostaglandines, intervenant dans les processus de l'inflammation, ou encore lors de la phagocytose.

Un radical libre peut se former selon deux types de processus chimiques:
- rupture homolytique d'une liaison chimique simple entre deux atomes d'une molécule, chacun emportant un électron de la liaison avec lui : 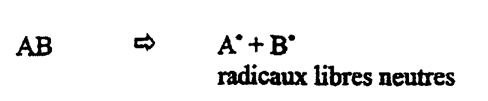
- transfert d'un électron (réaction d'oxydoréduction) entre deux molécules (un oxydant et un réducteur) qui donne naissance à deux radicaux chargés :

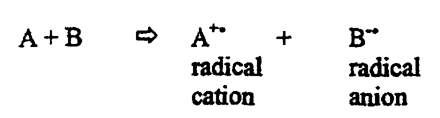

La réaction la plus courante à laquelle conduisent les radicaux libres en solution est l'arrachement d'un atome d'hydrogène sur un substrat présent dans le milieu, ce qui conduit à la formation d'un nouveau radical libre :

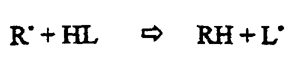

L'attaque par voie radicalaire initie des réactions en chaîne qui ne s'arrêtent que lorsque deux radicaux libres s'inactivent mutuellement

La peroxydation lipidique (lipoperoxydation) est un cas typique de réaction en chaîne induite par voie radicalaire.

L'oxydation des lipides membranaires aboutit à la formation de lipoperoxydes, qui se décomposent en différents produits de fragmentation dont certains sont très agressifs.

L'un des produits de fragmentation le plus important et le plus agressif est un aldéhyde, le malone-dialdéhyde (MDA). Porteur de deux radicaux libres, il manifeste une toxicité redoutable en pontant transversalement les protéines, les lipides intracellulaires et l'ADN.

Dans la toxicité des radicaux libres envers les systèmes biologiques, les espèces réactives dérivées de l'oxygène occupent une place prépondérante.

Les principales espèces radicalaires impliquées dans les processus biologiques sont les suivantes :

| | |
|---|---|
| ¹O2 | l'oxygène singulet |
| O2^{•-} | radical anion superoxyde qui induit la formation d'un radical beaucoup plus réactif: |
| OH^{•} | radical hydroxyle, oxydant très puissant |
| HOO^{•} | radical perhydroxyle (forme protonée de O2^{•-}) |
| LO^{•} | radical alkoxyle lipidique |
| LOO^{•} | radical peroxyle lipidique (L = groupement phospholipidique polyinsaturé) |

Les radicaux libres et la cascade de réactions en chaîne qu'ils provoquent au sein de l'organisme jouent un rôle très important dans le processus du vieillissement cutané.

Leur action destructrice au niveau des lipides membranaires entraîne la mort lente de la cellule.

Ils s'attaquent également au patrimoine génétique de la cellule : ADN et ARN, molécules essentielles aux réactions de synthèses protéiques.

Ils dégradent enfin les protéines, notamment les fibres de collagènes et d'élastine, dont les caractéristiques fonctionnelles sont gravement altérées.

De tout ceci résulte une peau abîmée, prématurément vieillie.

Or, le métabolisme cellulaire (notamment la respiration) est générateur de radicaux libres. C'est un phénomène tout à fait normal, que la cellule est à même de combattre car elle dispose de moyens de défense adaptés.

Dans certaines circonstances, cet équilibre harmonieux peut se rompre. Le système de protection naturel de la cellule n'est alors plus suffisamment efficace. Il existe de nombreuses causes déclenchant une rupture d'équilibre de la cellule vis-à-vis des radicaux libres :
- une mauvaise hygiène alimentaire
- l'exposition prolongée et répétée de l'organisme aux rayonnements ionisants (rayons X, U.V., bains de soleil excessifs)
- le mauvais usage de médicaments et l'abus de tabac ou d'alcool
- la pollution
- le stress, promoteur indirect de radicaux libres...

Il existe dans les cellules différents moyens de s'opposer aux radicaux libres et de les neutraliser : des agents protecteurs, des enzymes (superoxyde dismutase, glutathion peroxydase et catalane), certains oligo-éléments (zinc, sélénium) et vitamines (vitamines A-C et surtout E).

Mais un certain nombre de radicaux échappe à cette défense naturelle. Ceci peut entraîner, à la longue, un phénomène cumulatif qui intervient dans les processus d'involution et d'usure sénile.

Pour lutter contre ce phénomène, on a déjà proposé des composés antiradicalaires permettant d'aider les systèmes de défense naturels de la cellule pour permettre à l'organisme de retrouver un fonctionnement harmonieux. Ces composés, tout comme les molécules naturellement présentes au sein de la peau, vont, en raison d'affinités chimiques, physico-chimiques et électrochimiques beaucoup plus grandes pour les radicaux libres que pour les structures membranaires, former avec ceux-ci des produits stables non radicalaires.

Les substances antiradicalaires, selon leur nature, bloquent la réaction en chaîne à différents stades, stoppant ainsi, plus ou moins rapidement, l'agression radicalaire. Les lipides membranaires sont, de cette façon, préservés.

On rappelle en outre que chez les êtres vivants, les cellules se trouvent affectées par différents types de réactions oxydantes, dont les deux réactions suivantes :
- les réactions aboutissant à la production du radical hydroxyle OH^{•},
- celles provenant des hèmes tels que l'hémoglobine libre qui produit des radicaux de type oxoferryl.

Par ailleurs, on sait que le contenu en ATP (Adénosine TriPhosphate, substrat énergétique majeur de la cellule) dans les kératinocytes d'épiderme humain reflète en partie l'état énergétique des cellules : l'ATP, molécule de transfert et de stockage d'énergie est indispensable au métabolisme cellulaire.

On a constaté lors d'expérimentations que le contenu en ATP des kératinocytes chute de façon significative après exposition à des radiations telles que, par exemple, des irradiations ultraviolettes (UVB).

L'invention a donc plus particulièrement pour objet une composition qui :
- présente de très bonnes propriétés antiradicalaires de manière à obtenir une action anti-âge aussi bien pour des vieillissements naturels ou accidentels,
- assure une protection efficace contre les réactions aboutissant à la production de radicaux OH^{•} ou de radicaux de type oxoferryl,
- réduise la chute du contenu en ATP des kératinocytes après irradiation, de manière à obtenir un effet réparateur.

En vue d'obtenir ces résultats, elle se propose d'utiliser les propriétés du chrysanthellum indicum.

Inscrit sur la liste des plantes médicinales de la pharmacopée notamment pour ses actions hépatoprotectrice-hépatostimuiante, antilithiasique, anti-oedémateuse, anti-inflammatoire, le chrysanthellum indicum également appelé chrysanthellum americanum ou procumbens contient des acides phénylpropéniques, des flavonoïdes et des saponosides. Il conjugue des saponosides et des flavonoïdes d'une façon inhabituelle dans le monde végétal, avec une richesse exceptionnelle en flavonoïdes puisqu'il associe une flavone, une aurone, une chalcone et deux flavanones, association également peu fréquente dans le règne végétal.

De cette association tout à fait étonnante, résultent les nombreuses vertus du chrysanthellum indicum qui est un remède utilisé pour le traitement d'affections diverses, d'origine digestive, circulatoire, etc..., telles que :
- hépatites,
- colopathies,
- syndromes ictère-hémorragiques,
- lithiases biliaires et urinaires,
- pathologies cardio-vasculaires et circulatoires.

En particulier, le chrysanthellum indicum est préconisé dans les cas d'insuffisance biliaire, les séquelles d'hépatites, les intoxications alcooliques, les lithiases salivaires, rénales ou biliaires, les troubles entérocolitiques, les affections vasculaires, les perturbations du métabolisme lipidique.

Les études répertoriées montrent que les traitements à base de chrysanthellum s'effectuent par voie orale (tisane, extraits de plante en sirop, en gélule...) ou par voie intrapéritonéale, mais en aucun cas par voie topique.

Le seul document répertorié traitant de l'intérêt du chrysanthellum indicum dans des domaines autres que le domaine strictement médical est le brevet FR No 2 618 071 qui propose des compositions cosmétiques et dermatologiques renfermant entre 1 % et 10 % en poids d'extrait sec de chrysanthellum pour des applications telles que des shampooings et des lotions capillaires, des émulsions dermiques, des laits corporels, des rouges à lèvres, voire même des compositions cosmétiques sous formes d'aérosols.

Outre le fait que ce document n'évoque pas la prévention du vieillissement cutané, les concentrations d'utilisation qu'il préconise conduisent à des produits totalement inappropriés et même incompatibles avec un usage cosmétique normal. En effet, à ces concentrations, les émulsions à usage topique présentent notamment :
- Une coloration marron foncé qui provoque la teinture jaune moutarde intense de la peau à traiter ainsi que celle des doigts ayant servi à effectuer l'application. La forte intensité de cette teinture s'atténue au rinçage mais laisse subsister, après lavage, une coloration jaune foncé.
- Une mauvaise stabilité dans le temps : on constate, dès 24 heures, une concentration de l'eau dans le fond du récipient et un important relargage d'huile en surface.
- Une forte odeur d'extrait végétal.

Il s'avère que même dans l'hypothèse où l'on parviendrait à formuler, avec de telles concentrations d'extraits secs de chrysanthellum, un produit suffisamment stable, la coloration de ce produit resterait un problème majeur :
- Couleur trop intense, pas du tout conforme à ce qui peut être généralement accepté par un consommateur utilisateur de produits cosmétiques.
- Produit agissant comme une véritable teinture : la peau et les ongles sont inéluctablement teintés en jaune plus ou moins soutenu. De même, les tissus se trouvant au contact de ce produit sont teints de façon indélébile en une couleur jaune plus ou moins soutenue qui vire à l'orange après lavage avec les détergents habituels.

L'invention a donc plus particulièrement pour but la mise au point d'une composition administrable par voie topique, qui utilise les principes actifs du chrysanthellum indicum en matière de protection de la peau et de prévention du vieillissement cutané et qui résolve à la fois les problèmes de coloration et d'odeur précédemment évoqués.

L'invention parvient à ce résultat grâce à une composition comprenant un extrait de chrysanmellum indicum à faible concentration comportant de 0,0001 % à 0,05 %, soit de 1 µg/ml à 500 µg/ml d'équivalents d'extraits secs de chrysanmellum indicum.

Les résultats des essais qui ont été effectués montrent que dans cette plage de concentration, contre toute attente, le chrysanthellum exerce un puissant effet antiradicalaire et que, par ailleurs, les problèmes de coloration, d'odeur et de stabilité sont facilement résolus.

Lors de ces essais, les effets d'un extrait sec de chrysanthellum indicum sur la production de radicaux libres oxygénés ont été révélés à partir d'un modèle chimique consistant en une micro-émulsion en solution aqueuse de linoléate de sodium, les radicaux hydroxyles étant produits par radiolyse de l'eau.

Les radicaux oxoferryls ont été générés par le contact d'une solution d'hémoglobine avec la micro-émulsion préalablement ultrasoniqué pour produire en son sein dès traces de lipoperoxydes qui ont réagit avec l'hémoglobine en déclenchant une lipoperoxydation en chaîne.

Dans les deux cas, la lipoperoxydation a été suivie par application de la propriété que présentent les hydroperoxydes d'acide linoléique de se rompre en dégageant du pentane. Les réactions se sont déroulées en système clos, et des échantillons de gaz ont été prélevés et injectés dans un chromatographe en phase gazeuse, permettant le dosage du pentane, dont la quantité est proportionnelle aux lipoperoxydes formés. S'agissant d'un extrait organique dont on ignore la composition (ce qui ne permet pas un calcul de molarité), les résultats ont été exprimés en pourcentage de pentane par rapport aux témoins sans la substance (où se déroulera une peroxydation maximale). Il est évident que la baisse de production de pentane est proportionnelle à la chute des réactions de lipoperoxydation, donc à l'efficacité antioxydante du produit étudié.

La courbe d'inhibition de la substance étudiée a été comparée à celle d'antioxydants d'efficacité reconnue, ici l'acide urique en tant que antioxydant classique hydrosoluble et le BHT, liposoluble, protecteur des acides polyinsaturés à la concentration de 10⁻³ M.

### Evaluation du produit soumis :

La substance étudiée (extrait sec de chrysanthellum) a été dissoute dans un tampon phosphate à pH 7,4. Elle est donc hydrosoluble, ne laissant pas de trace de produit insoluble, et fournissant une solution transparente.

Cette étude a permis d'observer un très net effet protecteur de la substance étudiée, quel que soit le système de production des radicaux libres. Cet effet apparaît de façon surprenante aux très faibles doses et est déjà perceptible à 0,0015 µg/ml.

L'extrait de chrysanthellum sec étudié présente une efficacité anti-hydroxyle remarquable, puisque les extraits végétaux classiquement étudiés cessent d'être actifs au-dessous de 125 µg/ml. Son effet anti-oxoferryl est moins efficace tout en restant comparable à ceux des extraits végétaux les plus actifs connus, comme piégeurs de ce radical.

L'étude in vitro de l'effet de l'extrait sec de chrysanthellum indicum sur des cultures de kératinocytes humains ayant subi un stress aux ultraviolets B a permis de déceler un effet réparateur.

Dans cette étude, les cellules ont été soumises à différentes doses d'irradiation ultraviolettes (UVB). Ces rayonnements UVB induisent la formation de radicaux libres aux effets délétères sur le métabolisme cellulaire.

Après irradiation, les cellules ont été incubées pendant 24 heures avec l'extrait de chrysanthellum ou les molécules de référence (vitamine C et PABA, filtre UVB).

Le contenu en ATP des kératinocytes est mesuré aux temps T0 (avant irradiation), T1 (après irradiation) et T2 (24 heures après irradiation = durée d'incubation).

Le contenu en ATP des kératinocytes évalué immédiatement après irradiation (temps T1) a chuté dans tous les cas de figure. Cette diminution s'est accentuée au temps T2 dans le cas du témoin non traité.

L'effet du composé à l'essai et des molécules de référence sur le contenu cellulaire en ATP a été évalué au temps T2.

Cette étude a permis de constater qu'à une concentration de 0,003 %, l'extrait de chrysanthellum indicum augmente d'un facteur 1,8 (par rapport aux cellules irradiées non traitées) le contenu cellulaire en ATP des cellules irradiées aux doses de 0,945 et 1,89 J/cm².

Le chrysanthellum indicum permet aux kératinocytes lésés par des radiations UVB de récupérer une partie de leur taux initial d'ATP, plus rapidement que les kératinocytes lésés non traités.

Il a donc un effet tout à fait positif sur les cultures de kératinocytes humains ayant subi un stress UVB.

Cette observation, faite avec des concentrations faibles d'actif par rapport à celles utilisées généralement dans une formulation, est compatible avec les concentrations utilisées pour obtenir l'"effet réparateur" précédemment évoqué.

Il apparaît donc que par son action antiradicalaire et anti-lipoperoxydante, l'extrait de chrysanthellum indicum, sous quelque forme qu'il soit (extrait sec, solutions glycoliques...) est un agent de prévention du vieillissement cutané.

Il atténue les effets du temps et des agressions multiples que subit quotidiennement la peau, en neutralisant les radicaux libres produits par l'organisme ou en limitant leur production (production excessive lorsque la peau est soumise à des situations de "stress cellulaire").

Son utilisation est particulièrement recommandée pour la formulation de soins quotidiens à visée "anti-âge", de produits solaires et de produits après soleil.

Des exemples de formulation de compositions seront décrits ci-après, à titre d'exemples non limitatifs.

| Exemple I : Gel après soleil | | |
|---|---|---|
| A1 | Eau déminéralisée | QSP 100 % |
| A2 | Glycérine | de 1 à 5 % de préférence 3,00 % |
| B1 | Gélifiant acrylique | de 0,1 à 1 % de préférence 0,30 % |
| C1 | Triéthanolamine | de 0,1 à 1% de préférence 0,50 % |
| D1 | Conservateur antimicrobien | de 0,5 à 1% de préférence 0,55 % |
| E1 | Huile végétale on huile minérale | de 1 à 5 % de préférence 2,00 % |
| F1 | Parfum | de 0,1 à 1 % de préférence 0,15 % |
| H1 | Extrait de chrysanthellum en solution dans du butylène glycol et de l'eau (soit de 0,0001 % à 0,05 % d'extrait sec de chrysanthellum) | de 0,005 % à 2,5 % de préférence 0,50 % |

| Exemple II : Crème antiradicalaire | | |
|---|---|---|
| A1 | Stéarate de sorbitol | de 1 à 2 % de préférence 1,50 % |
| A2 | Stéarate de glycéryl et Polyéthylène glycol-100 | de 2 à 5 % de préférence 3,25 % |
| A3 | Huiles minérales et huiles végétales | de 10 à 20 % de préférence 13,50 % |
| B1 | Eau déminéralisée | QSP 100 % |
| B2 | Propylène glycol | de 1 à 5 % de préférence 2,50 % |
| C1 | Gélifiant acrylique | de 0,1 à 1 % de préférence 0,40 % |
| D1 | Triéthanolamine | de 0,1 à 1 % de préférence 0,40 % |
| E1 | Conservateur antimicrobien | de 0,5 à 1 % de préférence 0,80 % |
| F1 | Parfum | de 0,1 à 1 % de préférence 0,15 % |
| G1 | Gélifiant (par exemple du type SEPIGEL, commercialisé par la Société SEPPIC) | de 0,1 à 2 % de préférence 0,70 % |
| H1 | Extrait de chrysanthellum en solution dans du butylène glycol et de l'eau (soit de 0,0001 % à 0,05 % d'extrait sec de chrysanthellum) | de 0,005 % à 2,5 % de préférence 1,00 % |

| Exemple III : Lait solaire | | |
|---|---|---|
| A1 | Eau déminéralisée | QSP 100 % |
| A2 | Dioxyde de titane | de 0,1 à 2 % de préférence 0,20 % |
| A3 | Sorbitol | de 0,5 à 5 % de préférence 1 % |
| B1 | Triéthanolamine | de 0,1 à 1 % de préférence 0,30 % |
| C1 | Stéarate de glycéryl et Polyéthylène glycol-100 | de 1 à 5 % de préférence 2,00 % |
| C2 | Stéarate de glycéryl | de 0,1 à 1 % de préférence 0,30 % |
| C3 | Acide stéarique | de 0,5 à 5 % de préférence 1,00 % |
| C4 | Huiles végétales et huiles minérales | de 5 à 15 % de préférence 8,00 % |
| C5 | Filtre solaire (cinnamate) | de 0,5 à 10 % de préférence 5,00 % |
| C6 | Antioxydant | de 0,01 à 0,1 % de préférence 0,07 % |
| D1 | Gélifiant acrylique | de 0,1 à 1 % de préférence 0,10 % |
| E1 | Conservateur antimicrobien | de 0,5 à 1 % de préférence 0,80 % |
| F1 | Parfum | de 0.1 à 1% de préférence 0,40 % |
| G1 | Extrait de chrysanthellum en solution dans du propylène glycol et de l'eau (soit de 0,0001 % à 0,05 % d'extrait sec de chrysanthellum) | par exemple 5,00 % |

Dans ces exemples, l'extrait sec de chrysanthellum indicum est obtenu de façon classique en broyant la plante fraîche ou sèche jusqu'à obtention d'une poudre. On fait macérer cette poudre dans de l'eau éventuellement mélangée à de l'éthanol ou du méthanol. A partir de cette macération, on obtient, par lixiviation, un extrait qui, une fois lavé, est concentré puis évaporé jusqu'à siccité. On obtient une poudre soluble dans l'eau qui peut être utilisée en solution dans l'eau ou même en une solution hydroglycolique (butylène glycol ou propylène glycol) : En effet, dans les deux cas, on observe un effet protecteur identique vis-à-vis des processus peroxydatifs (inhibition du pentane produit par une émulsion d'acide linoléique). De même, on observe un effet protecteur identique sur la production de pentane induite par le radical hydroxyle. Cet extrait sec peut être également utilisé en solution dans un mélange eau/glycérol.

Eventuellement, la composition selon l'invention pourra comprendre un extrait sec de chrysanthellum indicum encapsulé, en phase continue aqueuse ou huileuse.

Bien entendu, les compositions selon l'invention pourront se présenter sous la forme d'émulsions simples ou multiples (crèmes ou laits eau/huile ou huile/eau, émulsions triples, micro-émulsions, émulsions à cristaux liquides), de gels aqueux ou huileux, de lotions aqueuses ou hydroalcooliques, de sticks, ou de poudres ou de tout système vectorisé (systèmes "à libération contrôlée" ou systèmes à "libération modulée"). Elles peuvent être utilisées par voie topique. De même, l'extrait de chrysanthellum indicum pourra être un extrait huileux.

## Revendications

1. Composition pour la prévention du vieillissement cutané,
**caractérisée en ce qu'**elle comprend un extrait de chrysanthellum indicum à faible concentration, ladite composition comportant de 0,0001 % à 0,05 % d'équivalents d'extraits secs de chrysanthellum indicum.

2. Composition selon la revendication 1,
**caractérisée en ce que** le susdit extrait est un extrait sec de chrysanthellum indicum.

3. Composition selon la revendication 1,
**caractérisée en ce que** le susdit extrait est un extrait huileux de chrysanthellum indicum.

4. Composition selon la revendication 1,
**caractérisée en ce que** le susdit extrait est en solution hydroglycolique.

5. Composition selon la revendication 1,
**caractérisée en ce que** le susdit extrait est en solution dans un mélange eau/glycérol.

6. Composition selon l'une des revendications 2 et 3,
**caractérisée en ce qu'**elle comprend un extrait de chrysanthellum indicum encapsulé en phase continue aqueuse ou huileuse.

7. Composition selon l'une des revendications précédentes,
**caractérisée en ce qu'**elle se présente sous la forme d'émulsions simples ou multiples.

8. Composition selon la revendication 7,
**caractérisée en ce qu'**elle se présente sous la forme d'une crème ou d'un lait eau/huile ou huile/eau.

9. Composition selon la revendication 7,
**caractérisée en ce qu'**elle se présente sous la forme d'une émulsion triple, d'une micro-émulsion ou d'une émulsion à cristaux liquides.

10. Composition selon l'une des revendications 1 à 6,
**caractérisée en ce qu'**elle se présente sous la forme de gels aqueux ou huileux.

11. Composition selon l'une des revendications 1 à 6,
**caractérisée en ce qu'**elle se présente sous la forme d'une lotion aqueuse ou hydroalcoolique.

12. Composition selon l'une des revendications 1 à 6,
**caractérisée en ce qu'**elle se présente sous la forme d'un stick.

13. Composition selon l'une des revendications 1 à 6,
**caractérisée en ce qu'**elle se présente sous la forme d'une poudre.

14. Composition selon l'une des revendications 1 à 6,
**caractérisée en ce qu'**elle se présente sous la forme d'un système vectorisé à libération contrôlée ou à libération modulée.

15. Composition selon l'une des revendications 1 à 6,
**caractérisée en ce qu'**elle se présente sous une forme utilisable par voie topique.

16. Composition pour soins après exposition solaire,
**caractérisée en ce qu'**elle comprend :
| | | |
|---|---|---|
| A1 | Eau déminéralisée | QSP 100 % |
| A2 | Glycérine | de 1 à 5 % |
| B1 | Gélifiant acrylique | de 0,1 à 1 % |
| C1 | Triéthanolamine | de 0,1 à 1 % |
| D1 | Conservateur antimicrobien | de 0,5 à 1 % |
| E1 | Huile végétale ou huile minérale | de 1 à 5 % |
| F1 | Parfum | de 0,1 à 1% |
| H1 | Extrait de chrysanthellum en solution dans du butylène glycol et de l'eau (soit de 0,0001 % à 0,05 % d'extrait sec de chrysanthellum) | de 0,005 % à 2,5 % |

17. Composition selon la revendication 16,
**caractérisée en ce qu'**elle comprend :
| | | |
|---|---|---|
| A1 | Eau déminéralisée | QSP 100% |
| A2 | Glycérine | 3,00 % |
| B1 | Gélifiant acrylique | 0,30 % |
| C1 | Triéthanolamine | 0,50 % |
| D1 | Conservateur antimicrobien | 0,55 % |
| E1 | Huile végétale ou huile minérale | 2,00 % |
| F1 | Parfum | 0,15% |
| H1 | Extrait de chrysanthellum en solution dans du butylène glycol et de l'eau (soit de 0,0001 % à 0,05 % d'extrait sec de chrysanthellum) | 0,50% |

18. Composition antiradicalaire,
**caractérisée en ce qu'**elle comprend :
| | | |
|---|---|---|
| A1 | Stéarate de sorbitol | de 1 à 2 % |
| A2 | Stéarate de glycéryl et Polyéthylène glycol-100 | de 2 à 5 % |
| A3 | Huiles minérales et huiles végétales | de 10 à 20 % |
| B1 | Eau déminéralisée | QSP 100% |
| B2 | Propylène glycol | de 1 à 5 % |
| C1 | Gélifiant acrylique | de 0,1 à 1 % |
| D1 | Triéthanolamine | de 0,1 à 1 % |
| E1 | Conservateur antimicrobien | de 0,5 à 1 % |
| F1 | Parfum | de 0,1 à 1 % |
| G1 | Gélifiant | de 0,1 à 2 % |
| H1 | Extrait de chrysanthellum en solution dans du butylène glycol et de l'eau (soit de 0,0001 % à 0,05 % d'extrait sec de chrysanthellum) | de 0,005 % à 2,5 % |

19. Composition selon la revendication 18,
**caractérisée en ce qu'**elle comprend :
| | | |
|---|---|---|
| A1 | Stéarate de sorbitol | 1,50 % |
| A2 | Stéarate de glycéryl et Polyéthylène glycol-100 | 3,25 % |
| A3 | Huiles minérales et huiles végétales | 13,50 % |
| B1 | Eau déminéralisée | QSP 100 % |
| B2 | Propylène glycol | 2,50 % |
| C1 | Gélifiant acrylique | 0,40 % |
| D1 | Triéthanolamine | 0,40 % |
| E1 | Conservateur antimicrobien | 0,80 % |
| F1 | Parfum | 0,15 % |
| G1 | Gélifiant | 0,70 % |
| H1 | Extrait de chrysauthellum en solution dans du butylène glycol et de l'eau (soit de 0,0001 % à 0,05 % d'extrait sec de chrysanthellum) | 1,00 % |

20. Lait solaire,
**caractérisé en ce qu'**il comprend :
| | | |
|---|---|---|
| A1 | Eau déminéralisée | QSP 100 % |
| A2 | Dioxyde de titane | de 0,1 à 2 % |
| A3 | Sorbitol | de 0,5 à 5 % |
| B1 | Triéthanolamine | de 0,1 à 1 % |
| C1 | Stéarate de glycéryl et Polyéthylène glycol-100 | de 1 à 5 % |
| C2 | Stéarate de glycéryl | de 0,1 à 1% |
| C3 | Acide stéarique | de 0,5 à 5 % |
| C4 | Huiles végétales et huiles minérales | de 5 à 15 % |
| C5 | Filtre solaire (cinnamate) | de 0,5 à 10 % |
| C6 | Antioxydant | de 0,01 à 0,1 % |
| D1 | Gélifiant acrylique | de 0,1 à 1 % |
| E1 | Conservateur antimicrobien | de 0,5 à 1 % |
| F1 | Parfum | de 0,1 à 1% |
| G1 | Extrait de chrysanthellum en solution dans du propylène glycol et de l'eau (soit de 0,0001 % à 0,05 % d'extrait sec de chrysanthellum) | 5,00% |

21. Lait solaire selon la revendication 20,
**caractérisé en ce qu'**il comprend :
| | | |
|---|---|---|
| A1 | Eau déminéralisée | QSP 100 % |
| A2 | Dioxyde de titane | 0,20 % |
| A3 | Sorbitol | 1 % |
| B1 | Triéthanolamine | 0,30 % |
| C1 | Stéarate de glycéryl et Polyéthylène glycol-100 | 2,00 % |
| C2 | Stéarate de glycéryl | 0,30 % |
| C3 | Acide stéarique | 1,00 % |
| C4 | Huiles végétales et huiles minérales | 8,00 % |
| C5 | Filtre solaire (cinnamate) | 5,00 % |
| C6 | Antioxydant | 0,07 % |
| D1 | Gélifiant acrylique | 0,10% |
| E1 | Conservateur antimicrobien | 0,80 % |
| F1 | Parfum | 0,40 % |
| G1 | Extrait de chrysanthellum en solution dans du propylène glycol et de l'eau (soit de 0,0001 % à 0,05 % d'extrait sec de chrysanthellum) | 5,00 % |

## Claims

1. Composition for preventing ageing of the skin, **characterized in that** it comprises an extract of Chrysanthellum indicum at low concentration, said composition comprising from 0.0001% to 0.05% of dry extract equivalents of Chrysanthellum indicum.

2. Composition according to Claim 1, **characterized in that** the abovementioned extract is a dry extract of Chrysanthellum indicum.

3. Composition according to Claim 1, **characterized in that** the abovementioned extract is an oily extract of Chrysanthellum indicum.

4. Composition according to Claim 1, **characterized in that** the abovementioned dry extract is in the form of an aqueous-glycolic solution.

5. Composition according to Claim 1, **characterized in that** the abovementioned dry extract is in the form of a solution in a water/glycerol mixture.

6. Composition according to either of Claims 2 and 3, **characterized in that** it comprises an encapsulated extract of Chrysanthellum indicum in an aqueous or oily continuous phase.

7. Composition according to one of the preceding claims, **characterized in that** it is in the form of simple or multiple emulsions.

8. Composition according to Claim 7, **characterized in that** it is in the form of a water/oil or oil/water cream or milk.

9. Composition according to Claim 7, **characterized in that** it is in the form of a triple emulsion, a microemulsion or an emulsion containing liquid crystals.

10. Composition according to one of Claims 1 to 6, **characterized in that** it is in the form of aqueous or oily gels.

11. Composition according to one of Claims 1 to 6, **characterized in that** it is in the form of an aqueous or aqueous-alcoholic lotion.

12. Composition according to one of Claims 1 to 6, **characterized in that** it is in the form of a stick.

13. Composition according to one of Claims 1 to 6, **characterized in that** it is in the form of a powder.

14. Composition according to one of Claims 1 to 6, **characterized in that** it is in the form of a controlled-release or modulated-release vectorized system.

15. Composition according to one of Claims 1 to 6, **characterized in that** it is in a form which can be used topically or orally.

16. Composition for care after exposure to the sun, **characterized in that** it comprises:
| | | |
|---|---|---|
| A1 | Demineralized water | qs 100% |
| A2 | Glycerol | from 1 to 5% |
| B1 | Acrylic gelling agent | from 0.1 to 1% |
| C1 | Triethanolamine | from 0.1 to 1% |
| D1 | Antimicrobial preserving agent | from 0.5 to 1% |
| E1 | Plant oil or mineral oil | from 1 to 5% |
| F1 | Fragrance | from 0.1 to 1% |
| H1 | Extract of chrysanthellum as a solution in butylene glycol and water (i.e. from 0.0001% to 0.05% of dry extract of chrysanthellum) | from 0.005% to 2.5% |

17. Composition according to Claim 16, **characterized in that** it comprises
| | | |
|---|---|---|
| A1 | Demineralized water | qs 100% |
| A2 | Glycerol | 3.00% |
| B1 | Acrylic gelling agent | 0.30% |
| C1 | Triethanolamine | 0.50% |
| D1 | Antimicrobial preserving agent | 0.55% |
| E1 | Plant oil or mineral oil | 2.00% |
| F1 | Fragrance | 0.15% |
| H1 | Extract of chrysanthellum as a solution in butylene glycol and water (i.e. from 0.0001% to 0.05% of dry extract of chrysanthellum) | 0.50% |

18. Anti-radical composition, **characterized in that** it comprises:
| | | |
|---|---|---|
| A1 | Sorbitol stearate | from 1 to 2% |
| A2 | Glyceryl stearate and polyethylene glycol-100 | from 2 to 5% |
| A3 | Mineral oils and plant oils | from 10 to 20% |
| B1 | Demineralized water | qs 100% |
| B2 | Propylene glycol | from 1 to 5% |
| C1 | Acrylic gelling agent | from 0.1 to 1% |
| D1 | Triethanolamine | from 0.1 to 1% |
| E1 | Antimicrobial preserving agent | from 0.5 to 1% |
| F1 | Fragrance | from 0.1 to 1% |
| G1 | Gelling agent | from 0.1 to 2% |
| H1 | Extract of chrysanthellum as solution in butylene glycol and water (i.e. from 0.0001% to 0.05% of dry extract of chrysanthellum) | from 0.005% to 2.5% |

19. Composition according to Claim 18, **characterized in that** it comprises:
| | | |
|---|---|---|
| A1 | Sorbitol stearate | 1.50% |
| A2 | Glyceryl stearate and polyethylene glycol-100 | 3.25% |
| A3 | Mineral oils and plant oils | 13.50% |
| B1 | Demineralized water | qs 100% |
| B2 | Propylene glycol | 2.50% |
| C1 | Acrylic gelling agent | 0.40% |
| D1 | Triethanolamine | 0.40% |
| E1 | Antimicrobial preserving agent | 0.80% |
| F1 | Fragrance | 0.15% |
| G1 | Gelling agent | 0.70% |
| H1 | Extract of chrysanthellum as solution in butylene glycol and water (i.e. from 0.0001% to 0.05% of dry extract of chrysanthellum) | 1.00% |

20. Antisun milk, **characterized in that** it comprises:
| | | |
|---|---|---|
| A1 | Demineralized water | qs 100% |
| A2 | Titanium dioxide | from 0.1 to 2% |
| A3 | Sorbitol | from 0.5 to 5% |
| B1 | Triethanolamine | from 0.1 to 1% |
| C1 | Glyceryl stearate and polyethylene glycol-100 | from 1 to 5% |
| C2 | Glyceryl stearate | from 0.1 to 1% |
| C3 | Stearic acid | from 0.5 to 5% |
| C4 | Plant oils and mineral oils | from 5 to 15% |
| C5 | Sunscreen (cinnamate) | from 0.5 to 10% |
| C6 | Antioxidant | from 0.01 to 0.1% |
| D1 | Acrylic gelling agent | from 0.1 to 1% |
| E1 | Antimicrobial preserving agent | from 0.5 to 1% |
| F1 | Fragrance | from 0.1 to 1% |
| G1 | Extract of chrysanthellum as a solution in propylene glycol and water (i.e. from 0.0001% to 0.05% of dry extract of chrysanthellum) | 5.00% |

21. Antisun milk according to Claim 20, **characterized in that** it comprises:
| | | |
|---|---|---|
| A1 | Demineralized water | qs 100% |
| A2 | Titanium dioxide | 0.20% |
| A3 | Sorbitol | 1% |
| B1 | Triethanolamine | 0.30% |
| C1 | Glyceryl stearate and polyethylene glycol-100 | 2.00% |
| C2 | Glyceryl stearate | 0.30% |
| C3 | Stearic acid | 1.00% |
| C4 | Plant oils and mineral oils | 8.00% |
| C5 | Sunscreen (cinnamate) | 5.00% |
| C6 | Antioxidant | 0.07% |
| D1 | Acrylic gelling agent | 0.10% |
| E1 | Antimicrobial preserving agent | 0.80% |
| F1 | Fragrance | 0.40% |
| G1 | Extract of chrysanthellum as a solution in propylene glycol and water (i.e. from 0.0001% to 0.05% of dry extract of chrysanthellum) | 5.00% |

## Patentansprüche

1. Zusammensetzung zur Verhinderung der Hautalterung, **dadurch gekennzeichnet, daß** sie einen Extrakt von Chrysanthellum indicum in einer geringen Konzentration von 0,0001% bis 0,05% Äquivalente Trockenextrakte von Chrysanthellum indicum umfaßt.

2. Zusammensetzung nach Anspruch 1, **dadurch gekennzeichnet, daß** der Extrakt ein Trockenextrakt von Chrysanthellum indicum ist.

3. Zusammensetzung nach Anspruch 1, **dadurch gekennzeichnet, daß** der Extrakt ein öliger Extrakt von Chrysanthellum indicum ist.

4. Zusammensetzung nach Anspruch 1, **dadurch gekennzeichnet, daß** der Extrakt in Hydroglykol-Lösung vorliegt.

5. Zusammensetzung nach Anspruch 1, **dadurch gekennzeichnet, daß** der Extrakt als Lösung in einer Mischung von Wasser/Glycerin vorliegt.

6. Zusammensetzung nach einem der Ansprüche 2 und 3, **dadurch gekennzeichnet, daß** sie einen in einer kontinuierlichen wäßrigen oder öligen Phase eingekapselten Extrakt von Chrysanthellum indicum umfaßt.

7. Zusammensetzung nach einem der vorhergehenden Ansprüche, **dadurch gekennzeichnet, daß** sie in Form von einfachen oder vielfachen Emulsionen vorliegt.

8. Zusammensetzung nach Anspruch 7, **dadurch gekennzeichnet, daß** sie in Form einer Creme oder Wasser/Öl- oder Öl/Wasser-Milch vorliegt.

9. Zusammensetzung nach Anspruch 7, **dadurch gekennzeichnet, daß** sie in Form einer Dreifachemulsion, einer Mikroemulsion oder einer Flüssigkristallemulsion vorliegt.

10. Zusammensetzung nach einem der Ansprüche 1 bis 6, **dadurch gekennzeichnet, daß** sie in Form von wäßrigen oder öligen Gelen vorliegt.

11. Zusammensetzung nach einem der Ansprüche 1 bis 6, **dadurch gekennzeichnet, daß** sie in Form einer wäßrigen oder hydroalkoholischen Lotion vorliegt.

12. Zusammensetzung nach einem der Ansprüche 1 bis 6, **dadurch gekennzeichnet, daß** sie in Form eines Stifts vorliegt.

13. Zusammensetzung nach einem der Ansprüche 1 bis 6, **dadurch gekennzeichnet, daß** sie in Form eines Puders vorliegt.

14. Zusammensetzung nach einem der Ansprüche 1 bis 6, **dadurch gekennzeichnet, daß** sie in Form eines Systems vorliegt, das auf eine kontrollierte Freisetzung oder eine modulierte Freisetzung gerichtet ist.

15. Zusammensetzung nach einem der Ansprüche 1 bis 6, **dadurch gekennzeichnet, daß** sie in einer Form vorliegt, die auf lokalem Weg oder oralem Weg verwendbar ist.

16. Zusammensetzung für die Pflege nach der Sonnenbestrahlung, **dadurch gekennzeichnet, daß** sie umfaßt:
| | | |
|---|---|---|
| A1 | demineralisiertes Wasser | QSP 100% |
| A2 | Glycerin | von 1 bis 5% |
| B1 | acrylisches Geliermittel | von 0,1 bis 1% |
| C1 | Triethanolamin | von 0,1 bis 1% |
| D1 | antimikrobielles Haltbarkeitsmittel | von 0,5 bis 1% |
| E1 | Pflanzenöl oder Mineralöl | von 1 bis 5% |
| F1 | Parfum | von 0,1 bis 1% |
| H1 | Extrakt von Chrysanthellum als Lösung in Butylenglykol und Wasser (0,0001% bis 0,05% Chrysanthellum-Trockenextrakt) | von 0,005 bis 2,5% |

17. Zusammensetzung nach Anspruch 16, **dadurch gekennzeichnet, daß** sie umfaßt:
| | | |
|---|---|---|
| A1 | demineralisiertes Wasser | QSP 100% |
| A2 | Glycerin | 3,00% |
| B1 | acrylisches Geliermittel | 0,30% |
| C1 | Triethanolamin | 0,50% |
| D1 | antimikrobielles Haltbarkeitsmittel | 0,55% |
| E1 | Pflanzenöl oder Mineralöl | 2,00% |
| F1 | Parfum | 0,15% |
| H1 | Extrakt von Chrysanthellum als Lösung in Butylenglykol und Wasser (0,0001% bis 0,05% Chrysanthellum-Trockenextrakt) | 0,50% |

18. Antiradikalische Zusammensetzung, **dadurch gekennzeichnet, daß** sie umfaßt:
| | | |
|---|---|---|
| A1 | Sorbitstearat | von 1 bis 2% |
| A2 | Stearat von Glycerin und Polyethylenglykol 100 | von 2 bis 5% |
| A3 | Mineralöle und Pflanzenöle | von 10 bis 20% |
| B1 | demineralisiertes Wasser | QSP 100% |
| B2 | Propylenglykol | von 1 bis 5% |
| C1 | acrylisches Geliermittel | von 0,1 bis 1% |
| D1 | Triethanolamin | von 0,1 bis 1% |
| E1 | antimikrobielles Haltbarkeitsmittel | von 0,5 bis 1% |
| F1 | Parfum | von 0,1 bis 1% |
| G1 | Geliermittel | von 0,1 bis 2% |
| H1 | Extrakt von Chrysanthellum als Lösung in Butylenglykol und Wasser (0,0001% bis 0,05% Chrysanthellum-Trockenextrakt) | von 0,005 bis 2,5% |

19. Zusammensetzung nach Anspruch 18, **dadurch gekennzeichnet, daß** sie umfaßt:
| | | |
|---|---|---|
| A1 | Sorbitstearat | 1,50% |
| A2 | Stearat von Glycerin und Polyethylenglykol 100 | 3,25% |
| A3 | Mineralöle und Pflanzenöle | 13,50% |
| B1 | demineralisiertes Wasser | QSP 100% |
| B2 | Propylenglykol | 2,50% |
| C1 | acrylisches Geliermittel | 0,40% |
| D1 | Triethanolamin | 0,40% |
| E1 | antimikrobielles Haltbarkeitsmittel | 0,80% |
| F1 | Parfum | 0,15% |
| G1 | Geliermittel | 0,70% |
| H1 | Extrakt von Chrysanthellum als Lösung in Butylenglykol und Wasser (0,0001% bis 0,05% Chrysanthellum-Trockenextrakt) | 100% |

20. Sonnenmilch, **dadurch gekennzeichnet, daß** sie umfaßt:
| | | |
|---|---|---|
| A1 | demineralisiertes Wasser | QSP 100% |
| A2 | Titandioxid | von 0,1 bis 2% |
| A3 | Sorbit | von 0,5 bis 5% |
| B1 | Triethanolamin | 0,1 bis 1% |
| C1 | Stearat von Glycerin und Polyethylenglykol 100 | von 1 bis 5% |
| C2 | Glycerylstearat | von 0,1 bis 1% |
| C3 | Stearinsäure | von 0,5 bis 5% |
| C4 | Pflanzenöle und Mineralöle | von 5 bis 15% |
| C5 | Sonnenfilter (Cinnamat) | von 0,5 bis 10% |
| C6 | Antioxidanz | von 0,01 bis 0,1% |
| D1 | acrylisches Geliermittel | von 0,1 bis 1% |
| E1 | antimikrobielles Haltbarkeitsmittel | von 0,5 bis 1% |
| F1 | Parfum | von 0,1 bis 1% |
| G1 | Extrakt von Chrysanthellum als Lösung in Propylenglykol und Wasser (0,0001% bis 0,05% Chrysanthellum-Trockenextrakt) | 5,00% |

21. Sonnenmilch nach Anspruch 20, **dadurch gekennzeichnet, daß** sie umfaßt:
| | | |
|---|---|---|
| A1 | demineralisiertes Wasser | QSP 100% |
| A2 | Titandioxid | 0,20% |
| A3 | Sorbit | 1% |
| B1 | Triethanolamin | 0,30% |
| C1 | Stearat von Glycerin und Polyethylenglykol 100 | 2,00% |
| C2 | Glycerylstearat | 0,30% |
| C3 | Stearinsäure | 1,00% |
| C4 | Pflanzenöle und Mineralöle | 8,00% |
| C5 | Sonnenfilter (Cinnamat) | 5,00% |
| C6 | Antioxidans | 0,07% |
| D1 | acrylisches Geliermittel | 0,10% |
| E1 | antimikrobielles Haltbarkeitsmittel | 0,80% |
| F1 | Parfum | 0,40% |
| G1 | Extrakt von Chrysanthellum als Lösung in Propylenglykol und Wasser (0,0001% bis 0,05% Chrysanthellum-Trockenextrakt) | 5,00% |
